# EUROPEAN PATENT APPLICATION

(11) **EP 4 834 309 A1**
(43) Date of publication of application: **04.11.2026**
(21) Application number: 24912403.3
(22) Date of filing: 12.12.2024
(51) Int. Cl.: A61K 8/04, A61K 8/19, A61K 8/39, A61K 8/73, A61Q 17/04

(54) **OIL-IN-WATER COSMETIC**

(30) Priority: 26.12.2023 JP 2023220009
(71) Applicant: Shiseido Company, Ltd., Chuo-ku Tokyo 104-0061 (JP)
(72) Inventor: MIZUGUCHI, Akari, Tokyo 104-0061 (JP); KANAYAMA, Kazutaka, Tokyo 104-0061 (JP)
(74) Representative: Ter Meer und Partner mbB
(86) International application number: PCT/JP2024/043983
(87) International publication number: WO 2025/142514

(57) **Abstract**

One aspect of an oil-in-water cosmetic according to the present invention includes (A) a powder, (B) a water-soluble alkyl-substituted polysaccharide derivative, and (C) polyglycerol polyricinoleate or a (polyglyceryl isostearate/dimer dilinoleic acid) copolymer.

## Description

### TECHNICAL FIELD

The present invention relates to oil-in-water cosmetics.

### BACKGROUND ART

Oil-in-water cosmetics have been widely used as a base for external preparations for skin, such as skin cosmetics or the like, which is directly applied onto the skin, because the oil-in-water cosmetics give a watery and fresh texture when applied onto the skin. In skin care and body care, protection of the skin from ultraviolet rays has been carried out routinely, and a fresh and moist feel upon use is also desired for UV care cosmetics. Thus, use of the oil-in-water cosmetic as a base is getting increasingly important.

Since UV care cosmetics are used not only in daily life but also for swimming in pools or the sea in summer, or in outdoor activities, such as sports involving perspiration, and the like, UV care cosmetics having high water resistance are desired. However, there is a problem that water resistance of the oil-in-water cosmetics is not sufficiently satisfactory.

In addition, a sunscreen cosmetic (a sunscreen cosmetic) that is one kind of the UV care cosmetic includes an ultraviolet-ray blocking agent, such as an ultraviolet-ray absorber, an ultraviolet-ray scattering agent, or the like, so that UV rays in the UVA region (320 nm to 400 nm) and in the UVB region (280 nm to 320 nm) are blocked from reaching the skin, and the skin is protected from damage by UV rays. In order to obtain a high ultraviolet-ray protection factor (SPF: Sun Protection Factor), it is important to improve dispersibility of the ultraviolet-ray blocking agent, and to stably disperse and blend the ultraviolet-ray blocking agent in the sunscreen cosmetic.

For example, it is disclosed that a cosmetic composition including an oil solution, in which fullerene serving as an ultraviolet-ray absorber is dispersed, a nonionic surfactant, a polyvalent alcohol, an oil solution, and water has excellent dispersion stability of the fullerene (see Patent Document 1).

### CITATION LIST

### PATENT DOCUMENT

Patent Document 1: Japanese Unexamined Patent Application Publication No. 2018-172362

### SUMMARY OF THE INVENTION

### TECHNICAL PROBLEM

In view of the above, an object of one aspect of the present invention is to provide an oil-in-water cosmetic having excellent water resistance, dispersibility, and ultraviolet-ray protection function, and providing a fresh and moist feel upon use.

### SOLUTION TO THE PROBLEM

In order to solve the above problems, one aspect of an oil-in-water cosmetic according to the present invention includes (A) a powder, (B) a water-soluble alkyl-substituted polysaccharide derivative, and (C) polyglycerol polyricinoleate or a (polyglyceryl isostearate/dimer dilinoleic acid) copolymer.

### EFFECTS OF THE INVENTION

According to one aspect of the present invention, there can be provided an oil-in-water cosmetic having excellent water resistance, dispersibility, and ultraviolet-ray protection function, and providing a fresh and moist feel upon use.

### DETAILED DESCRIPTION OF THE EMBODIMENTS

Embodiments of the present invention will be described in detail hereinafter. The embodiments are not limited to the following description, and can be appropriately modified without departing from the gist of the present invention. In the present specification, "to" indicating a numerical range means that numerical values before and after "to" are included as a lower limit value and an upper limit value, respectively, unless otherwise specified.

### (Oil-in-water cosmetic)

The oil-in-water cosmetic according to one embodiment includes (A) a powder, (B) a water-soluble alkyl-substituted polysaccharide derivative, and (C) polyglycerol polyricinoleate or a (polyglyceryl isostearate/dimer dilinoleic acid) copolymer, and may further include other components, as necessary.

The present inventors have conducted intensive studies and found that, in an oil-in-water cosmetic that can give a fresh and moist feel upon use, dispersibility of (A) a powder can be improved with the addition of (C) polyglycerol polyricinoleate or a (polyglyceryl isostearate/dimer dilinoleic acid), and this improvement of dispersibility of the (A) powder can achieve a more excellent ultraviolet-ray protection function and can further improve water resistance.

### <(A) Powder>

The powder (may be referred to as the "(A) component" hereinafter) is not particularly limited, and may be appropriately selected from powders used for cosmetics or the like. The powder is preferably a metal oxide, a powder having a hydrophobic surface, or the like. The above powders may be used alone or in combination of two or more.

A shape and size of the (A) component is not particularly limited, and may be appropriately selected according to the intended purpose. Examples of the shape of the (A) component include a spherical shape, a plate shape, a petal shape, a flake shape, a rod shape, a spindle shape, a needle shape, an irregular shape, and the like. Considering the size of the (A) component, the (A) component may be fine particles having an average particle size of approximately 1 µm or less, or may be of a pigment grade.

### <<Metal oxide>>

The metal oxide is not particularly limited, and may be appropriately selected according to the intended purpose. Examples of the metal oxide include titanium oxide, iron oxide, magnesium oxide, zinc oxide, calcium oxide, zirconium oxide, aluminum oxide, chromium oxide, barium sulfate, cerium oxide, and the like. The above metal oxides may be used alone or in combination of two or more. Moreover, the (A) component may be composite powder particles composed of two or more materials selected from the foregoing metal oxides.

### <<Powder having hydrophobic surface>>

The powder having the hydrophobic surface means a powder having a low affinity for water. Specifically, the powder having the hydrophobic surface means a powder to which hydrophobicity is imparted by subjecting the (A) component to a surface treatment using an oily component having a high affinity for the (A) component.

In the oil-in-water cosmetic according to the embodiment, the "hydrophobicity" is determined by evaluating according to the following method. Specifically, ion-exchanged water and the (A) component are placed in a transparent sealed container, and are stored at 50°C for one day. Then, a specialized evaluator performs visual observation, and evaluates that the (A) component is hydrophobic when the majority of the evaluation powder is present on the liquid surface of the ion-exchanged water.

Examples of the powder having the hydrophobic surface include: a hydrophobic treated powder obtained by performing a hydrophobization treatment on a base powder; an ultraviolet-ray scattering agent; and the like.

### -Hydrophobic treated powder-

The hydrophobic treated powder is a powder obtained by performing a hydrophobization treatment on a base powder.

The base powder is not particularly limited, and may be appropriately selected according to the intended purpose. Examples of the base powder include the above metal oxide, inorganic powders other than the above metal oxide, organic powders, silicone powders, inorganic pigments, organic pigments, metal powder pigments, and the like. The above base powders may be used alone or in combination of two or more.

Examples of the inorganic powders other than the metal oxide include powders, such as carbon black, magnesium carbonate, calcium carbonate, chromium hydroxide, aluminum silicate, aluminum hydroxide, magnesium silicate, aluminum magnesium silicate, mica (mica), synthetic mica, sericite, synthetic sericite, talc, kaolin, silicon carbide, barium sulfate, boron nitride, silica, zeolite, glass, and the like.

Examples of the organic powders include: metallic soap powders, polyamide resins (nylon), polymethylsilsesquioxane, crosslinked organopolysiloxane polymers, polystyrene, polyurethane, polyethylene, polypropylene, polyethylene terephthalate, polymethacrylate, such as polymethyl methacrylate, crosslinked polymethacrylate, such as methyl methacrylate crosspolymers, a composite of methyl polymethacrylate and polyisoprene, polyacrylate, acrylonitrile-methacrylic acid copolymer powders, polytetrafluoroethylene, (HDI/PPG/polycaprolactone) crosspolymers, styrene-acrylic acid copolymer resins, polytetrafluoroethylene, cellulose, and the like.

Examples of the metallic soap powders include calcium laurate, magnesium laurate, lithium laurate, barium laurate, zinc laurate, calcium myristate, magnesium myristate, lithium myristate, barium myristate, zinc myristate, calcium palmitate, magnesium palmitate, lithium palmitate, barium palmitate, zinc palmitate, calcium stearate, magnesium stearate, lithium stearate, barium stearate, zinc stearate, calcium isostearate, magnesium isostearate, lithium isostearate, barium isostearate, zinc isostearate, calcium arachinate, magnesium arachinate, lithium arachinate, barium arachinate, zinc arachinate, calcium behenate, magnesium behenate, lithium behenate, barium behenate, zinc behenate, calcium lignocerate, magnesium lignocerate, lithium lignocerate, barium lignocerate, zinc lignocerate, calcium palmitoleate, magnesium palmitoleate, lithium palmitoleate, barium palmitoleate, zinc palmitoleate, calcium oleate, magnesium oleate, lithium oleate, barium oleate, zinc oleate, calcium ricinoleate, magnesium ricinoleate, lithium ricinoleate, barium ricinoleate, zinc ricinoleate, calcium linoleate, magnesium linoleate, lithium linoleate, barium linoleate, zinc linoleate, calcium α-linolenate, magnesium α-linolenate, lithium α-linolenate, barium α-linolenate, zinc α-linolenate, calcium γ-linolenate, magnesium γ-linolenate, lithium γ-linolenate, barium γ-linolenate, zinc γ-linolenate, calcium arachidonate, magnesium arachidonate, lithium arachidonate, barium arachidonate, zinc arachidonate, calcium EPA, magnesium EPA, lithium EPA, barium EPA, zinc EPA, calcium DHA, magnesium DHA, lithium DHA, barium DHA, zinc DHA, calcium undecylenate, magnesium undecylenate, lithium undecylenate, barium undecylenate, zinc undecylenate, calcium salts of tall oil fatty acids, magnesium salts of tall oil fatty acids, lithium salts of tall oil fatty acids, barium salts of tall oil fatty acids, zinc salts of tall oil fatty acids, and the like.

Examples of the crosslinked organopolysiloxane polymers include: partially crosslinked methyl polysiloxane, such as (dimethicone/vinyl dimethicone) crosspolymers; partially crosslinked methylphenyl polysiloxane, such as (dimethicone/phenyl dimethicone) crosspolymers; partially crosslinked polyether-modified silicone, such as dimethicone copolyol crosspolymers; partially crosslinked alkyl-modified silicone; partially crosslinked alkyl-polyether-modified silicone, such as (lauryl dimethicone·PEG) crosspolymers; and the like. Specific examples of the crosslinked organopolysiloxane polymers include (dimethicone/vinyl dimethicone) crosspolymers, (dimethicone/phenylvinyl dimethicone) crosspolymers, (dimethicone/vinyl dimethicone/methicone) crosspolymers, (dimethicone/lauryl dimethicone) crosspolymers, (diphenyl dimethicone/vinyl dipheyl dimethicone/silsesquioxane) crosspolymers, (vinyl dimethicone/methicone silsesquioxane) crosspolymers, and the like.

Examples of the inorganic pigments include: inorganic white pigments, such as titanium dioxide and zinc oxide; inorganic red pigments, such as iron oxide (red iron oxide); inorganic yellow pigments, such as yellow iron oxide and yellow ocher; inorganic black pigments, such as black iron oxide and carbon black; inorganic green pigments, such as chromium oxide, chromium hydroxide, and cobalt titanate; inorganic blue pigments, such as ultramarine and Prussian blue; and the like.

Examples of the organic pigments include zirconium lakes, barium lakes, or aluminum lakes, such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404, Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, Blue No. 1, and the like.

Examples of the metal powder pigments include pearl pigments, aluminum powders, copper powders, and the like. Examples of the pearl pigments include titanium oxide-coated mica, colored titanium oxide-coated mica, bismuth oxychloride, fish scale foils, and the like.

A hydrophobizing agent to be applied to the base powder is not particularly limited, and may be appropriately selected from powders used for cosmetics and the like. Examples of the hydrophobizing agent include silicones, fluorines, amino acids, alkylalkoxysilanes, metallic soaps, ester oils, phospholipids, glycolipids, and the like. The above hydrophobizing agents may be used alone or in combination of two or more.

Examples of the silicones serving as the hydrophobizing agent include: silicone oils, such as methyl hydrogen polysiloxane, dimethyl polysiloxane (dimethicone), methylphenylpolysiloxane, methylcyclopolysiloxane, octamethylcyclotetrasiloxane, decamethylcyclopentasiloxane, dodecamethylcyclohexasiloxane, octamethyltrisiloxane, tetradecamethylhexasiloxane, dimethylsiloxane-methyl(polyoxyethylene)siloxane-methyl(polyoxypropylene)siloxane copolymers, dimethylsiloxane-methyl(polyoxyethylene)siloxane copolymers, dimethylsiloxane-methyl(polyoxypropylene)siloxane copolymers, dimethylsiloxane-methylcetyloxysiloxane copolymers, and dimethylsiloxane-methylstearoxysiloxane copolymers; alkylsilanes, such as methyltriethoxysilane, ethyltriethoxysilane, hexyltriethoxysilane, octyltriethoxysilane, and triethoxycaprylylsilane; fluoroalkylsilanes, such as trifluoromethylethyltrimethoxysilane and heptadecafluorodecyltrimethoxysilane; tetramethyltetrahydrogen cyclotetrasiloxane; trimethyltrihydrogen cyclotrisiloxane; and the like.

Examples of the fluorines serving as the hydrophobizing agent include perfluoroalkyl phosphoric acid esters, perfluoroalcohols, and the like.

Examples of the amino acids serving as the hydrophobizing agent include: amino acids; lipoamino acids; N-acylglutamic acid salts, such as dilauroyl glutamic acid lysine salt and stearoyl glutamic acid salt; N-acyllysine salt; and the like.

Examples of the alkylalkoxysilanes serving as the hydrophobizing agent include octyltriethoxysilane, octyltrimethoxysilane, and the like.

Examples of the metallic soaps serving as the hydrophobizing agent include stearic acids or salts of stearic acids, isostearic acids or salts of isostearic acids, and the like.

Examples of the ester oils serving as the hydrophobizing agent include polyglyceryl-2 tetraisostearate and the like.

Examples of the phospholipids serving as the hydrophobizing agent include lecithin, hydrogenated lecithin, alginic acid, and the like.

Examples of the glycolipids serving as the hydrophobizing agent include mannosylerythritol lipid and the like.

### -Ultraviolet-ray scattering agent-

The ultraviolet-ray scattering agent is not particularly limited, and may be appropriately selected according to the intended purpose. Examples of the ultraviolet-ray scattering agent include the materials listed as the metal oxide, the materials listed as the inorganic powders other than the metal oxide, and the like.

As the (A) component, a material appropriately synthesized by a method known in the related art may be used, or a commercially available product may be used.

An amount of the (A) component in the oil-in-water cosmetic according to one embodiment is not particularly limited, and may be appropriately selected according to the intended purpose. The amount of the (A) component relative to a total mass of the oil-in-water cosmetic is preferably 25 percent by mass or less, more preferably 20 percent by mass or less, and yet more preferably 10 percent by mass or less. A lower limit value of the amount of the (A) component is not particularly limited. The lower limit value of the amount of the (A) component relative to the total mass of the oil-in-water cosmetic is preferably 0.01 percent by mass or greater, more preferably 0.1 percent by mass or greater, and yet more preferably 1 percent by mass or greater. The upper limit value and the lower limit value of the amount of the (A) component can be appropriately combined. The combination of the upper limit value and lower limit value of the amount of the (A) component relative to the total mass of the oil-in-water cosmetic is preferably 0.01 percent by mass or greater and 25 percent by mass or less, more preferably 0.1 percent by mass or greater and 20 percent by mass or less, yet more preferably 0.1 percent by mass or greater and 20 percent by mass or less, and particularly preferably 1 percent by mass or greater and 10 percent by mass or less. It is preferable that the amount of the (A) component in the oil-in-water cosmetic according to one embodiment is 25 percent by mass or less in view of favorable dispersibility and a fresh and moist feel upon use. In addition, it is preferable that the amount of the (A) component in the oil-in-water cosmetic according to one embodiment is 0.01 percent by mass or greater in view of an excellent ultraviolet-ray protection function.

### <(B) Water-soluble alkyl-substituted polysaccharide derivative>

The water-soluble alkyl-substituted polysaccharide derivative (may be referred to as the "(B) component" hereinafter) refers to a polysaccharide substituted with an alkyl group in the oil-in-water cosmetic according to one embodiment. The (B) component functions as a water-soluble thickening agent in the oil-in-water cosmetic.

The "alkyl group" in the (B) component is not particularly limited, and may be appropriately selected according to the intended purpose, as long as the alkyl group can impart hydrophobicity to the (B) component. The alkyl group is preferably an alkyl group including 1 to 35 carbon atoms, more preferably an alkyl group including 5 to 30 carbon atoms, yet more preferably an alkyl group including 6 to 26 carbon atoms, yet even more preferably an alkyl group including 10 to 25 carbon atoms, and particularly preferably an alkyl group including 14 to 22 carbon atoms. In addition, the alkyl group in the (B) component may be directly bonded to the polysaccharide, or may be bonded to the polysaccharide as an indirect substituent.

The "polysaccharide" in the (B) component is a compound in which a plurality of monosaccharides are bonded together. The polysaccharide is not particularly limited, and may be appropriately selected according to the intended purpose. Examples of the polysaccharide include sclerotium gum, gum arabic, polysaccharides produced by Alcaligenes, cellulose, guar gum, carrageenan, hyaluronic acid, chondroitin sulfate, Polianthes Tuberosa polysaccharide, Tremella Fuciformis polysaccharide, locust beam gum, dextrin, chitosan, insulin, starch, and the like.

The polysaccharide may be modified for bonding with the alkyl group. Examples of the modification in the polysaccharide include methylcellulose, ethylcellulose, propylcellulose, butylcellulose, hydroxymethylcellulose, hydroxyethylcellulose, hydroxypropylcellulose, hydroxypropylmethylcellulose, carboxymethylcellulose, carboxyethylcellulose, hydroxypropylstarch phosphate, and the like. Among the above modifications, the polysaccharide may have one modification, or two or more modifications. Moreover, the polysaccharide may be hydroxypolysaccharide including one or more hydroxyl groups.

The number of monosaccharides constituting the polysaccharide is not particularly limited, and may be appropriately selected according to the intended purpose, as long as the (B) component is water soluble. The number of monosaccharides constituting the polysaccharide is preferably 100 to 100,000, more preferably 100 to 10,000, and yet more preferably 1,000 to 4,000.

Among the above, the (B) compound is preferably a hydrophobically modified alkyl cellulose (may be referred to as a "hydrophobic-modified alkyl cellulose" hereinafter). The hydrophobic-modified alkyl cellulose is a compound in which a long-chain alkyl group serving as a hydrophobic group is introduced into a water-soluble cellulose ether derivative, and is represented by the following general formula (I). Among compounds represented by the general formula (1), the (B) compound is more preferably a hydrophobic-modified alkyl cellulose, which is hydrophobically modified with an alkyl group including 14 to 22 carbon groups. 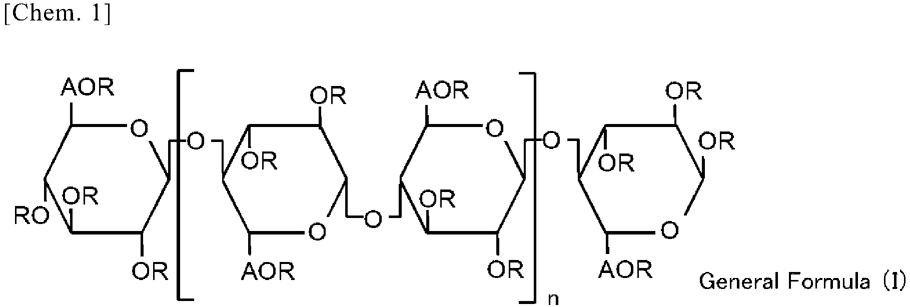 (In the general formula (I), each R is independently at least one group selected from the group consisting of a hydrogen atom, an alkyl group including 1 to 4 carbon atoms, -[CH₂CH(CH₃)O]ₘ-H (in the formula, m is an integer of 1 to 5, and preferably an integer of 1 to 3), -CH₂CH₂OH, and -CH₂CH(OH)CH₂OR' (in the formula, R' is an alkyl group including 14 to 22 carbon atoms). However, at least one R includes -CH₂CH(OH)CH₂OR'. Moreover, A is -(CH₂)_{q}- (in the formula, q is an integer of 1 to 3, and preferably 1), and n is an integer of 100 to 10,000, and preferably an integer of 500 to 5,000).

In the general formula (I), "each" R being "independently" means that two or more of R may be the same or different.

As the (B) component, a component appropriately synthesized by a method known in the related art may be used, or a commercial product may be used. An example of a production method in the case where the (B) component is the hydrophobic-modified alkyl cellulose represented by the general formula (I) will be described. The hydrophobic-modified alkyl cellulose can be obtained by bringing a compound for introducing a long-chain alkyl group including 14 to 22 carbon atoms into contact with a water-soluble cellulose ether derivative serving as a base in the presence of an alkali catalyst.

Examples of the water-soluble cellulose ether derivative serving as the base include methylcellulose (R is a hydrogen atom or a methyl group), ethylcellulose (R is a hydrogen atom or an ethyl group), propylcellulose (R is a hydrogen atom or a propyl group), butylcellulose (R is a hydrogen atom or a butyl group), hydroxypropylcellulose (R is a hydrogen atom or a hydroxypropyl group (-[CH₂CH(CH₃)O]ₘ-H (in the formula, m is an integer of 1 to 5, and is preferably an integer of 1 to 3))), hydroxypropylmethylcellulose (R is a hydrogen atom, a methyl group, or a hydroxypropyl group), and the like. Among the above water-soluble cellulose ether derivatives, the water-soluble cellulose ether derivative is preferably hydroxypropylmethylcellulose. In the case where the hydroxypropylmethylcellulose is used, R in the general formula (I) is any of four groups selected from a hydrogen atom, a methyl group, -[CH₂CH(CH₃)O]ₘ-H, and -CH₂CH(OH)CH₂OR', q of A is 1, and A is a methylene group.

Examples of the compound for introducing the long-chain alkyl group including 14 to 22 carbon atoms include a long-chain alkyl glycidyl ether represented by the following general formula (II). 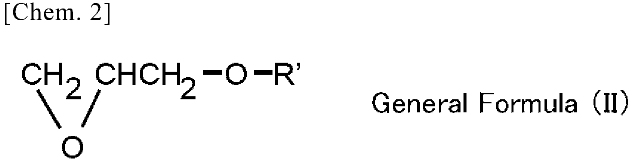 (In the general formula (II), R' is an alkyl group including 14 to 22 carbon atoms).

In the general formula (II), R' is an alkyl group including 14 to 22 carbon atoms, preferably an alkyl group including 14 to 20 carbon atoms, and more preferably a stearyl group (-C₁₈H₃₇) including 18 carbon atoms. When R' is an alkyl group including 14 to 22 carbon atoms, the resultant hydrophobic-modified alkyl cellulose can impart favorable emulsification stability.

An amount of -CH₂CH(OH)CH₂OR' to be introduced into the hydrophobic-modified alkyl cellulose is not particularly limited, but the amount of -CH₂CH(OH)CH₂OR' relative to a total mass of the hydrophobic-modified alkyl cellulose is preferably approximately 0.1 percent by mass to 5.0 percent by mass. In order to satisfy the preferable amount of -CH₂CH(OH)CH₂OR', the (B) component may be produced by appropriately selecting a molar ratio, a reaction time, a type of the alkali catalyst at the time of the reaction between the water-soluble cellulose ester derivative and the long-chain alkyl glycidyl ether. After the reaction, a purification step, such as neutralization, filtration, washing, drying, sieving, or the like of the reaction product, may be performed.

A weight average molecular weight of the hydrophobic-modified alkyl cellulose is not particularly limited, and may be appropriately selected according to the intended purpose. The weight average molecular weight of the hydrophobic-modified alkyl cellulose is preferably 100,000 to 1,000,000, more preferably 300,000 to 800,000, and yet more preferably 550,000 to 750,000.

Examples of a commercially available product of the (B) compound include hydrophobic-modified alkyl cellulose (weight average molecular weight (Mw) of 300,000 to 500,000, SANGELOSE (registered trademark) 60 series), hydrophobic-modified alkyl cellulose (weight average molecular weight (Mw) of 700,000 to 900,000, SANGELOSE (registered trademark) 90 series) (both manufactured by DAIDO CHEMICAL CORPORATION), and the like. The above commercially available products may be used alone or in combination of two or more.

An amount of the (B) component in the oil-in-water cosmetic according to one embodiment is not particularly limited, and may be appropriately selected according to the intended purpose. The amount of the (B) component to the total mass of the oil-in-water cosmetic is preferably 0.01 percent by mass to 1 percent by mass, more preferably 0.05 percent by mass to 0.5 percent by mass, and yet more preferably 0.1 percent by mass to 0.3 percent by mass. When the amount of the (B) component in the oil-in-water cosmetic according to one embodiment is 0.01 percent by mass to 1 percent by mass, sufficient emulsification stability can be obtained.

### <(C) Polyglycerol polyricinoleate or (polyglyceryl isostearate/dimer dilinoleic acid) copolymer>

Either the polyglycerol polyricinoleate or the (polyglyceryl isostearate/dimer dilinoleic acid) copolymer (may be referred to as a "(C) component" hereinafter) is used as the (C) component in the cosmetic according to one embodiment.

### <<Polyglycerol polyricinoleate>>

The polyglycerol polyricinoleate is an ester formed between a condensate (condensed ricinoleic acid) in which ricinoleic acids, which are higher fatty acids including 18 carbon atoms, are bonded together with an ester bond, and polyglycerin-6.

The polyglycerol polyricinoleate may be appropriately synthesized by a method known in the related art, or a commercial product of the polyglycerol polyricinoleate may be used. Examples of the commercial product of the polyglycerol polyricinoleate include: NIKKOL Decaglyn PR-20 (manufactured by Nikko Chemicals Co., Ltd.); SY-Glyster CR-310, CR-500, and CRS-75 (all manufactured by Sakamoto Yakuhin Kogyo Co., Ltd.); and the like.

### <<(Polyglyceryl isostearate/dimer dilinoleic acid) copolymer>>

The (polyglyceryl isostearate/dimer dilinoleic acid) copolymer is a copolymer of polyglyceryl-2 isostearate and dilinoleic acid.

The (polyglyceryl isostearate/dimer dilinoleic acid) copolymer may be appropriately synthesized by a method known in the related art, or a commercially available product of the (polyglyceryl isostearate/dimer dilinoleic acid) copolymer may be used. Examples of the commercially available product of the (polyglyceryl isostearate/dimer dilinoleic acid) copolymer include HAILUCENT ISDA (manufactured by KOKYU ALCOHOL KOGYO CO., LTD.).

An amount of the (C) component in the oil-in-water cosmetic according to one embodiment is not particularly limited, and may be appropriately selected according to the intended purpose. The amount of the (C) component relative to the total mass of the oil-in-water cosmetic is preferably 0.5 percent by mass to 10 percent by mass, more preferably 2 percent by mass to 6 percent by mass, and yet more preferably 3 percent by mass to 4 percent by mass. When the amount of the (C) component in the oil-in-water cosmetic according to one embodiment is 0.5 percent by mass or greater, favorable dispersibility of the (A) component is achieved. When the amount of the (C) component in the oil-in-water cosmetic according to one embodiment is 10 percent by mass or less, favorable water resistance is achieved.

### <Mass ratio [(C)/(A)]>

A mass ratio [(C)/(A)] of an amount (percent by mass) of the (C) polyglycerol polyricinoleate or (polyglyceryl isostearate/dimer dilinoleic acid) copolymer to an amount (percent by mass) of the (A) powder is not particularly limited, and may be appropriately selected according to the intended purpose. The mass ratio [(C)/(A)] is preferably 0.1 or greater and 2 or less, more preferably 0.1 or greater and 1 or less, and yet more preferably 0.5 or greater and 1 or less. When the mass ratio [(C)/(A)] is 0.1 or greater, favorable dispersibility of the (A) component is achieved. When the mass ratio [(C)/(A)] is 2 or less, favorable water resistance and ultraviolet-ray protection function are achieved.

### <Other components>

The cosmetic according to one embodiment may include other components in addition to the (A) component, the (B) component, and the (C) component. The other components are not particularly limited, and may be appropriately selected from known components used for cosmetics. Examples of the other components include oil components, higher alcohols, glycols, glycerins, sugar alcohols, saccharides, solvents, surfactants, moisturizing agents, ultraviolet-ray absorbers other than the (A) component, aqueous thickening agents other than the (C) component, dispersants, preservatives, fragrances, various drugs, and the like.

Amounts of the other components in the oil-in-water cosmetic according to one embodiment are not particularly limited, and may be appropriately selected according to the intended purpose, as long as the effects of the oil-in-water cosmetic are not impaired.

### <<Oil components>>

Examples of the oil components include ester oils, oils and fats, wax, hydrocarbon oils, higher fatty acids, higher alcohols, silicone oils, oil-soluble drugs, and the like. The above oil components may be used alone or in combination of two or more.

The ester oils are not particularly limited, and may be appropriately selected according to the intended purpose. Examples of the ester oils include glyceryl tri(2-ethylhexanoate) (glyceryl triisooctanoate), pentaerythrityl tetra(2-ethylhexanoate) (pentaerythrityl tetraoctanoate), glyceryl triisopalmitate, octyl isopalmitate, isopropyl isostearate, isostearyl isostearate, isocetyl isostearate, hexyl isostearate, myristyl isostearate, isocetyl octanoate, cetyl isooctanoate, isostearyl octanoate, isodecyl isononanoate, octyldodecyl dimethyloctanoate, oleyl myristate, isostearyl erucate, isocetyl stearate, octyl stearate, isostearyl palmitate, isocetyl palmitate, octyl palmitate, isostearyl myristate, isocetyl myristate, octyldodecyl myristate, decyl myristate, isopropyl palmitate, isopropyl myristate, trimethylol propane triisostearate, neopentyl glycol dicaprate, dioctyl sebacate, octyldodecyl 12-stearoyloxystearate, dioctyldodecyl stearoylglutamate, dioctyl adipate, diisostearyl malate, octyl methoxycinnamate, octyldodecyl lactate, isostearyl lactate, octyl para-dimethylaminobenzoate, and the like.

Examples of the oils and fats include: vegetable oils and fats, such as avocado oil, camellia oil, turtle oil, macadamia nut oil, corn oil, mink oil, olive oil, rapeseed oil, sesame oil, persic oil, wheat germ oil, sasanqua oil, castor oil, linseed oil, safflower oil, cottonseed oil, perilla oil, soybean oil, peanut oil, tea seed oil, torreya nucifera seed oil, rice bran oil, China wood oil, Japanese tung oil, jojoba oil, germ oil, cacao butter, coconut oil, hardened coconut oil, palm oil, palm kernel oil, and Japan wax kernel oil; animal oils and fats, such as egg yolk oil, horse fat, tallow, mutton tallow, hydrogenated beef tallow, beef bone fat, and neatsfoot oil; and the like.

Examples of the wax include Japan wax, beeswax, candelilla wax, cotton wax, carnauba wax, bayberry wax, insect wax, spermaceti wax, montan wax, bran wax, lanolin, Ceiba pentandra wax, lanolin acetate, liquid lanolin, sugarcane wax, lanolin fatty acid isopropyl ester, hexyl laurate, reduced lanolin, jojoba wax, hard lanolin, shellac wax, and the like.

Examples of the hydrocarbon oils include straight-chain, branched-chain, or volatile hydrocarbon oils. Specific examples of the hydrocarbon oils include liquid paraffin, squalane, isoparaffin, α-olefin oligomers, polybutene, microcrystalline wax, hydrogenated polydecene, isohexadecane, and the like.

Examples of the higher fatty acids include lauric acid, myristic acid, palmitic acid, stearic acid, behenic acid, oleic acid, 12-hydroxystearic acid, undecylenic acid, lanolin fatty acids, isostearic acid, linoleic acid, linolenic acid, eicosapentaenoic acid, and the like.

Examples of the higher alcohols include alcohols including 6 or more carbon atoms, and the like. Specific examples of the higher alcohols include straight-chain or branched-chain higher alcohols, such as lauryl alcohol, cetyl alcohol, stearyl alcohol, behenyl alcohol, myristyl alcohol, oleyl alcohol, cetearyl alcohol, monostearyl glycerin ether (batyl alcohol), 2-decyltetradecynol, 2-octyldodecanol, lanolin alcohol, cholesterol, phytosterol, hexyldodecanol, isostearyl alcohol, octyldodecanol, and the like.

Examples of the silicone oils include volatile cyclic silicone, volatile dimethyl polysiloxane, methyl phenyl silicone, and the like.

Examples of the oil-soluble drugs include fat-soluble vitamins, such as vitamin A, retinol, retinol palmitate, benzyl nicotinate, dl-α-tocopherol nicotinate, vitamin D2, tocopherol, and the like.

### <<Higher alcohols>>

Examples of the higher alcohols include: saturated straight-chain monovalent alcohols, such as dodecanol (lauryl alcohol), tridodecanol, tetradodecanol (myristyl alcohol), pentadecanol, hexadecanol (cetyl alcohol), heptadecanol, octadecanol (stearyl alcohol), nonadecanol, icosanol (arachidyl alcohol), heneicosanol, docosanol (behenyl alcohol), tricosanol, tetracosanol (carnaubyl alcohol), pentacosanol, and hexacoasnol (ceryl alcohol); unsaturated monovalent alcohols, such as elaidyl alcohol; and the like.

### <<Glycols>>

Examples of the glycols include propylene glycol, dipropylene glycol, 1,3-butylene glycol, 1,4-butylene glycol, diethylene glycol, triethylene glycol, polyethylene glycol, and the like. The above glycols may be used alone or in combination of two or more.

### <<Glycerins>>

Examples of the glycerins include glycerin, diglycerin, polyglycerin, and the like. The above glycerins may be used alone or in combination of two or more.

### <<Sugar alcohols>>

Examples of the sugar alcohols include sorbitol, mannitol, maltitol, xylitol, erythritol, and the like. The above sugar alcohols may be used alone or in combination of two or more.

### <<Saccharides>>

Examples of the saccharides include fructose, glucose, galactose, maltose, lactose, trehalose and the like. The above saccharides may be used alone or in combination of two or more.

### <<Solvents>>

Examples of the solvents include water, lower alcohols, and the like. Examples of the lower alcohols include alcohols including 5 or less carbon atoms. Specific examples of the lower alcohols include methanol, ethanol, propyl alcohol, isopropyl alcohol, 2-amino-2-methyl-1-propanol, 2-amino-2-methyl-1,3-propane diol, diisopropyl sebacate, and the like. The above solvents may be used alone or in combination of two or more.

### <<Surfactants>>

Examples of the surfactants include anionic surfactants, cationic surfactants, nonionic surfactants, amphoteric surfactants, and the like. The above surfactants may be used alone or in combination.

Examples of the anionic surfactants include: a soap base; fatty acid soaps, such as sodium laurate and sodium palmitate; higher alkyl sulfate, such as sodium lauryl sulfate and potassium lauryl sulfate; alkyl ether sulfates, such as triethanol amine polyoxyethylene (may be abbreviated as "POE" hereinafter) lauryl sulfate and sodium POE lauryl sulfate; N-acylsarcosine acid, such as sodium lauroylsarcosine; higher fatty acid amide sulfonic acid, such as sodium N-myristoryl-N-methyltaurate and sodium cocoyl methyl taurate; phosphate salts, such as POE stearyl ether phosphate; sulfosuccinates, such as sodium monolauroylmonoethanolamide POE sulfosuccinate, and sodium lauryl polypropylene glycol sulfosuccinate; alkylbenzene sulfonates, such as sodium linear decylbenzene sulfonate and triethanol amine linear dodecylbenzene sulfonate; N-acylgultamate, such as disodium N-stearoylglutamate and monosodium N-stearoylglutamate; higher fatty acid ester sulfate salts, such as sodium hydrogenated glyceryl cocoate sulfate; sulfonated oil, such as sulfonated castor oil; POE alkyl ether carboxylic acid; POE alkyl allyl ether carboxylate; higher fatty acid ester sulfonate; secondary alcohol sulfate salts; higher fatty acid alkanolamide sulfate salts; sodium lauryl monoethanolamide succinate; sodium casein; and the like.

Examples of the cationic surfactants include: alkyl trimethyl ammonium salts, such as stearyl trimethyl ammonium chloride and lauryl trimethyl ammonium chloride; dialkyl dimethyl ammonium salts, such as distearyl dimethyl ammonium chloride; alkyl pyridinium salts, such as cetyl pyridinium chloride; alkyl quaternary ammonium salts; alkyldimethylbenzyl ammonium salts; alkylisoquinolinium salts; dialkylmorpholinium salts; POE alkyl amine; alkyl amine salts; polyamine fatty acid derivatives; amyl alcohol fatty acid derivatives; benzalkonium chloride; and the like.

Examples of the nonionic surfactants include polyethylene glycol fatty acid esters, glyceryl polyoxyethylene fatty acids, polyoxyethylene-methyl polysiloxane copolymers, fatty acid polyoxyethylene sorbitan, polyoxyethylene alkyl ethers, maltitol hydroxyaliphatic alkyl ethers, alkylated polysaccharides, alkyl glucosides, sucrose fatty acid esters, glyceryl polyoxyethylene hydrogenated castor oil, polyoxyethylene sorbit fatty acid esters, polyoxyethylene-polyoxypropylene block copolymers, tetrapolyoxyethylene/tetrapolyoxypropylene-ethylene diamine condensates, polyoxyethylene-bees wax/lanolin derivatives, alkanol amides, polyoxyethylene-propylene glycol fatty acid esters, polyoxyethylene-alkyl amine, polyoxyethylene-fatty acid amine, alkylethoxydimethylamine oxide, trioleyl phosphoric acid, and the like.

Examples of the amphoteric surfactants include: imidazoline-based amphoteric surfactants, such as 2-cocoyl-2-imidazoliniumhydroxide-1-carboxyethyloxy disodium salt; betaine-based surfactants, such as amidobetaine and sulfobetaine; and the like.

### <<Moisturizing agents>>

Examples of the moisturizing agent include polyethylene glycol (may be abbreviated as "PEG" hereinafter), sodium chondroitin sulfate, sodium hyaluronate, sodium lactate, glucosamine, cyclodextrin, and the like. Moreover, the above glycols, the above glycerins, the above sugar alcohols, and the above saccharides may also be used as the moisturizing agents. The above moisturizing agents may be used alone or in combination of two or more.

### <<Aqueous thickening agents other than (B) component>>

Examples of the aqueous thickening agents include natural water-soluble polymers, semisynthetic water-soluble polymers, synthetic water-soluble polymers, inorganic thickening agents, and the like. The above aqueous thickening agents may be alone or in combination of two or more.

Examples of the natural water-soluble polymers include: plant-based polymers, such as gum arabic, gum tragacanth, galactan, guar gum, carrageenan, pectin, quince seed (quince) extracts, agar, and brown algae powders; microbial polymers, such as xanthan gum, dextran, pullulan, and succinoglycan; animal-based polymers, such as collagen, casein, albumin, and gelatin; and the like.

Examples of the semisynthetic water-soluble polymers include: starch-based polymers, such as carboxymethyl starch and methylhydroxy starch; alginic acid-based polymers, such as sodium alginate and propylene glycol alginate; and the like.

Examples of the synthetic water-soluble polymers include: vinyl polymers, such as polyvinyl alcohol, polyvinyl acetate, polyvinyl methyl ether, polyvinyl pyrrolidone, vinyl pyrrolidone-vinyl acetate copolymers, and carboxy vinyl polymers; acrylic polymers, such as sodium polyacrylate, polyethyl acrylate, polyacrylamide, acrylic acid-alkyl methacrylate copolymers (e.g., (acrylate/alkyl (C10-30) acrylate) crosspolymers), alkanol amine polyacrylate, copolymers of alkyl methacrylate and dimethylaminoethyl methacrylate, poly(2-acrylaide-2-methylpropane sulfonic acid), polymethacryloyloxytrimethyl ammonium, (ammonium acryloyldimethyltaurate /VP) copolymers, (dimethylacrylamide/Na acryloyldimethyltaurate) copolymers, (dimethylacrylamide/Na acryloyldimethyltaurate) crosspolymers; and the like.

Examples of the inorganic thickening agents include bentonite, laponite, hectorite, aluminum magnesium silicate, silicic anhydride, and the like.

### <<Ultraviolet-ray absorbers other than (A) component>>

Examples of the ultraviolet-ray absorbers other than the (A) component include: benzoic acid-based ultraviolet-ray absorbers, such as p-aminobenzoic acid; anthranilic acid-based ultraviolet-ray absorbers, such as methyl anthranilate; salicylic acid-based ultraviolet-ray absorbers, such as octyl salicylate; cinnamic acid-based ultraviolet-ray absorbers, such as isopropyl p-methoxycinnamate, octyl p-methoxycinnamate, and ethylhexyl methoxycinnamate; ultraviolet-ray absorbers, such as urocanic acid and ethyl urocanate; benzophenone-based ultraviolet-ray absorbers, such as 2-hydroxy-4-methoxybenzophenone and dihydroxybenzophenone; benzotriazole-based ultraviolet-ray absorbers; 2-phenylbenzimidazole-5-sulfonic acid; and the like. The above ultraviolet-ray absorbers may be used alone or in combination of two or more.

### <<Dispersants>>

Examples of the dispersants include sorbitan sesquiisostearate, isostearic acid, palmitic acid, silicone-based surfactants, and the like. The above dispersants may be used alone or in combination of two or more.

### <<Preservatives>>

Examples of the preservative include benzoic acid, salicylic acid, p-oxybenzoic acid ester (e.g., methylparaben, ethylparaben, butylparaben, etc.), sorbic acid, p-chloro-m-cresol, hexachlorophene, benzalkonium chloride, chlorohexidine chloride, trichlorocarbanilide, photosensitizers, phenoxyethanol, and the like. The above preservatives may be used alone or in combination of two or more.

### <<Fragrances>>

Examples of the fragrance include: natural fragrances obtained from animals or plants; synthesized fragrances produced by a chemical synthesis method; formulated fragrances that are any mixture of the foregoing; and the like. Specific examples of the fragrances include acetybenol, anisaldehyde, anethole, amyl acetate, amyl salicylate, allylamyl glycolate, allyl caproate, aldehyde C6-C20, ambretride, ambretlide, ambroxan, ionone, ISOE Super, Eugenol, auranthiol, galaxolide, calone, geraniol, geranyl acetate, Sandaroa, Santalol, Sandera, Cyclamenaldehyde, cis-3-hexenyl acetate, cis-3-hexenol, citral, citronellyl acetate, citronellol, cineol, dihydromyrcenol, Jasmolactone, cinamic alcohol, cinamic aldehyde, styraryl acetate, cedryl acetate, cedrol, damascon, damassenone, decalactone, terpinyl acetate, terpineol, tonalid, tonalide, tripral, nerol, bacdanol, vanillin, hydroxycitronellal, phenylethyl acetate, phenylethyl alcohol, hexyl salicylate, betiberyl acetate, hedion, heliotropin, helional, belt fix, benzyl acetate, benzyl salicylate, benzyl benzoate, pentalid, bornyl acetate, myol, musk ketone, methyl anthranilate, methyldihydrojasmonate, yarayra, lime oxide, linalyl acetate, linalool, limonene, liral, lilial, rose oxide, rosinol, angelica oil, anise oil, almoise oil, basil oil, bay oil, bergamot oil, calamus oil, camphor oil, cananga oil, cardamon oil, cassia oil, cedarwood oil, celery oil, chamomile oil, cinnamon oil, clove oil, coriander oil, cumin oil, dill oil, elemi oil, estragon oil, eucalyptus oil, fennel oil, fenugreek oil, galbanum oil, geranium oil, ginger oil, grapefruit oil, guaiacwood oil, hiba oil, cypress oil, juniper berry oil, lavandin oil, lavender oil, lemon oil, lime oil, mandarin oil, ziram oil, mimosa oil, peppermint oil, spearmint oil, mill oil, myrtle oil, nutmeg oil, oakmoss oil, olibanum oil, opoponax oil, orange oil, parsley oil, patchouli oil, pepper oil, perilla oil, petigrain oil, neroli oil, orange flower oil, pimento oil, allspice oil, pine oil, rose oil, rosemary oil, clary sage oil, sage oil, sandalwood oil, styrax oil, tagetes oil, thyme oil, tuberose oil, valerian oil, vetiver oil, violet leaf oil, wintergreen oil, wormwood oil, ylang-ylang oil, yuzu oil, cassia absolute, genet absolute, hyacinth absolute, immortelle absolute, jasmine absolute, jonquil absolute, narcissus absolute, rose absolute, violet leaf absolute, benzoin, and the like. The above fragrances may be used alone or in a combination of two or more.

### <<Drugs>>

Examples of the drugs include ascorbic acid (vitamin C), tranexamic acid, kojic acid, ellagic acid, arbutin, alcoxylsalicylic acid, nicotinamide, glycyrrhetinic acid, tocopherol, retinol, salts of the foregoing, derivatives of the foregoing, and the like. The above drugs may be used alone or in combination of two or more.

Examples of the derivatives include sodium L-ascorbate, magnesium L-ascorbate, L-ascorbic acid glucoside, 2-O-ethyl-L-ascorbic acid, 3-O-ethyl-L-ascorbic acid, sodium 4-methoxysalicylate, potassium 4-methoxysalicylate, dipotassium glycyrrhizinate, stearyl glycyrrhizinate, tocopherol acetate, retinol acetate, retinol palmitate, and the like.

### [Production method]

A production method for the oil-in-water cosmetic is not particularly limited, and can be appropriately selected from known production methods for oil-in-water emulsified compositions. As a specific example of the production method for the oil-in-water cosmetic according to one embodiment, water, the (B) component, and aqueous components among the other components are mixed to prepare an aqueous phase. Next, the (A) component, the (C) component, and water-insoluble components among the other components are mixed to prepare an oil phase. Next, the oil phase is added to the aqueous phase, followed by stirring, to thereby produce the oil-in-water cosmetic according to one embodiment.

### [Application]

An application of the oil-in-water cosmetic according to one embodiment is not particularly limited, and may be appropriately selected according to the intended purpose. Examples of the application of the oil-in-water cosmetic include; cosmetics for skincare, such as lotions, creams, milky lotions, and serums; make-up cosmetics, such as foundations and cosmetic base; sunscreen cosmetics; tanning cosmetics; skin cleansing cosmetics, such as face wash and make-up removers; ointments; and the like. In addition, as the application of the oil-in-water cosmetic according to one embodiment, the oil-in-water cosmetic may have an embodiment in which the oil-in-water cosmetic is impregnated in a base material, such as packs. Among the above applications, the application of the oil-in-water cosmetic according to one embodiment is preferably a sunscreen cosmetic because the oil-in-water cosmetic has excellent water resistance, dispersibility, and ultraviolet-ray protection function, and gives a fresh and moist feel upon use.

### Examples

The embodiments will be more specifically described through Examples and Comparative Examples hereinafter, but the embodiments are not limited by Examples and Comparative Examples. In Examples and Comparative Examples, each blending amount is a value in "percent by mass" relative to a total mass of the oil-in-water cosmetic and is a value converted to a pure component basis. In addition, the mass ratio [(C)/(A)] is determined to the first decimal place by rounding at the second decimal place.

### (Examples 1 to 4)

The components were mixed according to the composition and amounts (percent by mass) presented in Table 1 to prepare an oil-in-water cosmetic of each of Examples 1 to 4 according to the common procedure. Specifically, first, the (B) component and water-soluble components among other components were dissolved in water to prepare an aqueous phase. Next, the (A) component, the (C) component, and water-insoluble components among the other components were mixed to prepare an oil phase. Finally, the oil phase was added to the aqueous phase, followed by stirring, thereby obtaining an oil-in-water cosmetic of each of Examples 1 to 4.

### (Comparative Examples 1 to 3)

The components were mixed according to the composition and amounts (percent by mass) presented in Table 2 to prepare an oil-in-water cosmetic of each of Comparative Examples 1 to 3 according to the common procedure. Specifically, first, the (B) component and water-soluble components among the other components were dissolved in water to prepare an aqueous phase. Next, the (A) component, a comparative component of the (C) component, and water-insoluble components among the other components were mixed to prepare an oil phase. Finally, the oil phase was added to the aqueous phase, followed by stirring, thereby obtaining an oil-in-water cosmetic of each of Comparative Examples 1 to 3.

The oil-in-water cosmetics of Examples 1 to 4 and Comparative Examples 1 to 3 were evaluated on "water resistance", "dispersibility", "ultraviolet-ray protection function", and "fresh and moist feel" by the following methods. The evaluation results are presented in Table 1 and Table 2 below.

### <Evaluation of water resistance>

Each of the oil-in-water cosmetics of Examples 1 to 4 and Comparative Examples 1 to 3 was used as a measurement sample, and the water resistance of the sample was evaluated by the following method. The measurement sample in an amount of 2 mg/cm² was dropped on a plate (S plate) (5 cm × 5 cm V-groove PMMA plate, SPFMASTER-PA01, manufactured by Fuji Kasei Co., Ltd.), applied with a finger for 60 seconds, and dried for 15 minutes. Subsequently, the absorbance of the plate on which the measurement sample had been added was measured at 280 nm to 500 nm by a spectrophotometer (U-3500 self-recording spectrophotometer, manufactured by Hitachi, Ltd.). Then, the measured plate was sufficiently immersed in water having a hardness of 50 to 500, and was stirred in water at 300 rpm for 30 minutes by a three-one motor. Then, the plate was dried at room temperature (25°C ± 5°C) for approximately 15 minutes to approximately 30 minutes until water droplets disappeared from the surface of the plate, and absorbance was measured again. From the integrated values of the absorbance (Abs) at 280 nm to 500 nm before and after the water bath, a rate of change in the absorbance (Abs) before and after the water bath was calculated according to the following equation (1). The higher the value of the rate of change in the absorbance before and after the water bath, the smaller the amount of the measurement sample dissolved into the water bath, indicating higher water resistance. Therefore, the rate of change in absorbance before and after the water bath was evaluated as "water resistance". [Equation (1)] 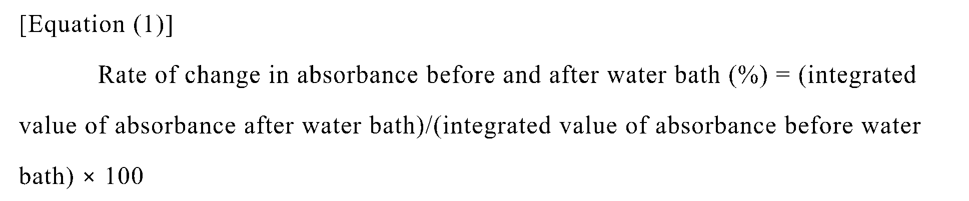 Rate of change in absorbance before and after water bath (%) = (integrated value of absorbance after water bath)/(integrated value of absorbance before water bath) × 100

### <Evaluation of dispersibility>

Each of the oil-in-water cosmetics of Examples 1 to 4 and Comparative Examples 1 to 3 was used as a measurement sample, and the dispersibility was evaluated by the following method. The measurement sample was placed in a sample inlet of a stress-controlled rheometer (manufactured by Anton Paar GmbH), and shear stress at the shear rate of 0.1 S⁻¹ to 1,000 S⁻¹ was measured at 30°C using a cone-plate type jig (CP50-1). The shear velocity was determined according to the following equation (2), and a flow curve representing a relationship between the shear velocity and the shear rate was obtained. A viscosity attenuation rate was calculated according to the following equation (3) using the viscosity values η (10/S) and η (1,000/S) at 10 S⁻¹ and 1,000 S⁻¹ on the obtained flow curve. The smaller the value of the viscosity attenuation rate, the smaller the degree of the viscosity change accompanying the increase in the viscosity rate, and thus can be evaluated as having high dispersibility of the powder. Therefore, the dispersibility of the powder was evaluated based on the following judgement criteria according to the calculated viscosity attenuation ratio. [Equation (2)] $Apparent viscosity \left(η\right) = shear stress \left(τ\right) / shear rate \left(γ\right)$
[Math. 1] [Equation (3)] $Viscosity attenuation rate = \frac{η \left(10/S\right) - η \left(1,000/S\right)}{η \left(10/S\right)}$

### -Judgement criteria of dispersibility-

A: viscosity attenuation rate being 0.2 or less
B: viscosity attenuation rate being greater than 0.2 and 0.4 or less
C: viscosity attenuation rate being greater than 0.4 and 0.7 or less
D: viscosity attenuation rate being greater than 0.7

### <Evaluation of ultraviolet-ray protection function>

Each of the oil-in-water cosmetics of Examples 1 to 4 and Comparative Examples 1 to 3 was used as a measurement sample, and the ultraviolet-ray protection function was evaluated by the following method. The measurement sample in an amount of 2 mg/cm² was dropped on a plate (S plate) (5 cm × 5 cm V-groove PMMA plate, SPFMASTER-PA01, manufactured by Fuji Kasei Co., Ltd.), applied with a finger for 60 seconds, and dried for 15 minutes. Subsequently, the absorbance of the plate on which the measurement sample had been added was measured at 280 nm to 500 nm by a spectrophotometer (U-3500 self-recording spectrophotometer, manufactured by Hitachi, Ltd.). A sample for measurement uncoated with the measurement sample was used as a control, and absorbance (Abs) was calculated according to the following equation (4). A higher value of absorbance (Abs) at 290 nm can be evaluated as having the high ultraviolet-ray protection function. The ultraviolet-ray protection function was evaluated based on the following judgement criteria according to the calculated absorbance [Equation (4)] $Abs = - log \left({T / T}_{0}\right)$ (In the equation (4), "T" is transmittance of the measurement sample, and "T₀" is transmittance of the control.)

### -Judgement criteria of ultraviolet-ray protection function-

A: the value of absorbance (Abs) at 290 nm being 1 or greater
B: the value of absorbance (Abs) at 290 nm being less than 1

### <Evaluation of fresh and moist feel>

Each of the oil-in-water cosmetics of Examples 1 to 4 and Comparative Examples 1 to 3 was evaluated for a fresh and moist feel when the oil-in-water cosmetic was applied onto the skin by the following method. A panel of 20 cosmetic evaluation specialists evaluated a fresh and moist feel of the oil-in-water cosmetic when the oil-in-water cosmetic was applied onto their arms based on the following evaluation criteria. The average point value of the points from the panel of 20 cosmetic evaluation specialists was calculated, and was determined based on the following judgement criteria.

### -Evaluation criteria of fresh and moist feel-

2 points: a fresh and moist feel being recognized
1 point: a fresh and moist feel being slightly recognized
0 points: no watery and fresh feel being recognized

### -Judgement criteria of fresh and moist feel-

A: the average point value was 1.75 points or greater, and no cosmetic evaluation specialist panelist gave the evaluation of 0 points
B: the average point value was 1.5 points or greater and less than 1.75 points, and two or less cosmetic evaluation specialist panelists gave the evaluation of 0 points.
C: the average point value was 1.0 points or greater and less than 1.5 points.
D: the average point value was less than 1.0 points.

**[Table 1]**

| Component name | | Ex. 1 | Ex. 2 | Ex. 3 | Ex. 4 |
|---|---|---|---|---|---|
| (A) component | hydrophobization-treated titanium oxide | 4.0 | 4.0 | 4.0 | 4.0 |
| (B) component | stearoxy hydroxypropylmethyl cellulose | 0.3 | 0.3 | 0.3 | 0.3 |
| (C) component | (polyglyceryl-2 isostearate/dimer dilinoleic acid) copolymer | 3.0 | - | 1.5 | 0.5 |
| | polyglyceryl-6 polyricinoleate | - | 3.0 | - | - |
| Comparative component of (C) component | polyglyceryl-2 diisostearate | - | - | - | - |
| | sorbitan sesquiisostearate | - | - | - | - |
| | polyhydroxystearic acid | - | - | - | - |
| | dimethicone | 1.0 | 1.0 | 1.0 | 1.0 |
| | ethylhexyl methoxycinnamate | 7.0 | 7.0 | 7.0 | 7.0 |
| | diisopropyl sebacate | 7.0 | 7.0 | 7.0 | 7.0 |
| | methylphenylpolysiloxane | 1.0 | 1.0 | 1.0 | 1.0 |
| Other components | isohexadecane | 4.0 | 4.0 | 4.0 | 4.0 |
| | (dimethylacrylamide/Na acryloyldimethyltaurate)crosspolymer | 0.2 | 0.2 | 0.2 | 0.2 |
| | PEG-6 | 7.0 | 7.0 | 7.0 | 7.0 |
| | water | balance | balance | balance | balance |
| | ethanol | 10.0 | 10.0 | 10.0 | 10.0 |
| | glycerin | 4.0 | 4.0 | 4.0 | 4.0 |
| Total (mass%) | | 100.0 | 100.0 | 100.0 | 100.0 |
| Mass ratio [(C)/(A)] | | 0.8 | 0.8 | 0.4 | 0.1 |
| Evaluation results | Water resistance [Absorbance change rate (%) before and after water bath] | 68 | 78 | 51 | 44 |
| | Dispersibility | A | A | A | A |
| | UV-ray protection function | A | A | B | B |
| | Fresh and moisture feel | A | A | A | A |

**[Table 2]**

| Component name | | Comp. Ex. 1 | Comp. Ex. 2 | Comp. Ex. 3 |
|---|---|---|---|---|
| (A) component | hydrophobization-treated titanium oxide | 4.0 | 4.0 | 4.0 |
| (B) component | stearoxy hydroxypropylmethyl cellulose | 0.3 | 0.3 | 0.3 |
| (C) component | (polyglyceryl-2 isostearate/dimer dilinoleic acid) copolymer | - | - | - |
| | polyglyceryl-6 polyricinoleate | - | - | - |
| Comparative component of (C) component | polyglyceryl-2 diisostearate | 3.0 | - | - |
| | sorbitan sesquiisostearate | - | 3.0 | - |
| | polyhydroxystearic acid | - | - | 3.0 |
| | dimethicone | 1.0 | 1.0 | 1.0 |
| | ethylhexyl methoxycinnamate | 7.0 | 7.0 | 7.0 |
| | diisopropyl sebacate | 7.0 | 7.0 | 7.0 |
| | diphenylsiloxy phenyl trimethicone | 1.0 | 1.0 | 1.0 |
| | isohexadecane | 4.0 | 4.0 | 4.0 |
| Other component | (dimethylacrylamide/Na acryloyldimethyltaurate) crosspolymer | 0.2 | 0.2 | 0.2 |
| | PEG-6 | 7.0 | 7.0 | 7.0 |
| | water | balance | balance | balance |
| | ethanol | 10.0 | 10.0 | 10.0 |
| | glycerin | 4.0 | 4.0 | 4.0 |
| Total (mass%) | | 100.0 | 100.0 | 100.0 |
| Mass ratio [(C)/(A)] | | - | - | - |
| Evaluation results | Water resistance [Absorbance change rate (%) before and after water bath] | 42 | 50 | 58 |
| | Dispersibility | C | C | C |
| | UV-ray protection function | A | B | B |
| | Fresh and moist feel | A | A | A |

The components used in Examples and Comparative Examples are as follows.
Hydrophobization-treated titanium oxide: hydrogen dimethicone-treated titanium oxide (manufactured by SAKAI CHEMICAL INDUSTRY CO., LTD.) Stearoxyhydroxypropylmethylcellulose: SANGELOSE90L (manufactured by DAIDO CHEMICAL CORPORATION)
(Polyglyceryl isostearate-2/dimer dilinoleic acid) copolymer: HAILUCENT ISDA, (manufactured by KOKYU ALCOHOL KOGYO CO., LTD.)
Polyglyceryl-6 polyricinoleate: SY-Glyster CR-75 (manufactured by Sakamoto Yakuhin Kogyo Co., Ltd.)
Dimethicone: dimethicone having viscosity of 1.35 mm²/s to 1.65 mm²/s at 25°C Polyglyceryl-2 diisostearate: WOGEL-18DV (manufactured by Matsumoto Fine Chemical Co., Ltd.)
Sorbitan sesquiisostearate: Estemol 182V (manufactured by The Nisshin OilliO Group, Ltd.)
Polyhydroxystearic acid: HS oligomer 600 (manufactured by Nikko Chemicals Co., Ltd.)

For example, embodiments of the present invention include the following.
<1> An oil-in-water cosmetic, including:
   (A) a powder;
   (B) a water-soluble alkyl-substituted polysaccharide derivative; and
   (C) polyglycerol polyricinoleate or a (polyglyceryl isostearate/dimer dilinoleic acid) copolymer.
<2> The oil-in-water cosmetic according to <1>,
   wherein the (A) powder is a metal oxide.
<3> The oil-in-water cosmetic according to <1> or <2>,
   wherein the (A) powder is a powder having a hydrophobic surface.
<4> The oil-in-water cosmetic according to <3>,
   wherein the powder having the hydrophobic surface is an ultraviolet-ray scattering agent.
<5> The oil-in-water cosmetic according to any one of <1> to <4>,
   wherein the (B) water-soluble alkyl-substituted polysaccharide derivative is a hydrophobic-modified alkyl cellulose.
<6> The oil-in-water cosmetic according to any one of <1> to <5>,
   wherein a mass ratio [(C)/(A)] of an amount of the (C) polyglycerol polyricinoleate or (polyglyceryl isostearate/dimer dilinoleic acid) copolymer to an amount of the (A) powder is 0.1 or greater and 2 or less.
<7> The oil-in-water cosmetic according to any one of <1> to <6>,
   wherein the oil-in-water cosmetic is a sunscreen cosmetic.

As described above, the present invention has been described based on the specific embodiments and examples, but these embodiments and examples are merely presented as examples, and the present invention is not limited by the above embodiments and examples. The above embodiments can be implemented in various other forms, and various combinations, omissions, substitutions, additions, changes, and the like can be made without departing from the gist of the invention. These embodiments and modifications are included in the scope and gist of the invention, and are included in the invention described in the scope of claims and the equivalent scope of the claims.

The present international application claims priority based on Japanese Patent Application No. 2023-220009, filed on December 26, 2023, and the entire contents of Japanese Patent Application No. 2023-220009 are incorporated herein by reference.

## Claims

1. An oil-in-water cosmetic, comprising:
(A) a powder;
(B) a water-soluble alkyl-substituted polysaccharide derivative; and
(C) polyglycerol polyricinoleate or a (polyglyceryl isostearate/dimer dilinoleic acid) copolymer.

2. The oil-in-water cosmetic according to claim 1,
wherein the (A) powder is a metal oxide.

3. The oil-in-water cosmetic according to claim 1,
wherein the (A) powder is a powder having a hydrophobic surface.

4. The oil-in-water cosmetic according to claim 3,
wherein the powder having the hydrophobic surface is an ultraviolet-ray scattering agent.

5. The oil-in-water cosmetic according to claim 1,
wherein the (B) water-soluble alkyl-substituted polysaccharide derivative is a hydrophobic-modified alkyl cellulose.

6. The oil-in-water cosmetic according to claim 1,
wherein a mass ratio [(C)/(A)] of an amount of the (C) polyglycerol polyricinoleate or (polyglyceryl isostearate/dimer dilinoleic acid) copolymer to an amount of the (A) powder is 0.1 or greater and 2 or less.

7. The oil-in-water cosmetic according to claim 1,
wherein the oil-in-water cosmetic is a sunscreen cosmetic.
